(19) Europäisches Patentamt
European Patent Office
Office européen des brevets

(11) Numéro de publication : **0 117 196**
**B1**

# (12) FASCICULE DE BREVET EUROPÉEN

(45) Date de publication du fascicule du brevet : **30.07.86**

(51) Int. Cl.⁴ : **C 07 D213/89**, C 07 D405/12, A 61 K 31/44

(21) Numéro de dépôt : **84400289.9**

(22) Date de dépôt : **13.02.84**

(54) **Nouveaux dérivés de bétaines n-iminopyridinium, leur préparation et leur application en tant que médicaments.**

(30) Priorité : **15.02.83 FR 8302380**

(43) Date de publication de la demande : **29.08.84 Bulletin 84/35**

(45) Mention de la délivrance du brevet : **30.07.86 Bulletin 86/31**

(84) Etats contractants désignés : **AT BE CH DE FR GB IT LI LU NL SE**

(56) Documents cités : **FR-A- 2 167 659**

(73) Titulaire : **PROVESAN S.A.**
**1, place St Gervais**
**CH-1211 Genève (CH)**

(72) Inventeur : **Esteve Soler, José**
**Via Augusta 244**
**Barcelone (ES)**

(74) Mandataire : **Ahner, Francis et al**
**CABINET REGIMBEAU 26, avenue Kléber**
**F-75116 Paris (FR)**

**EP 0 117 196 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés de bétaïnes N-imino-pyridinium, également dénommés sels internes d'hydroxyde d'aminopyridinium, leur préparation et leur application en tant que médicaments.

L'état de la technique peut par exemple être illustré par FR-A-2 167 659 qui décrit des dérivés de pyridinium définis de façon très générique. Ces dérivés sont en outre présentés comme substances acaricides et non pas comme médicaments.

Les nouveaux dérivés, objet de la présente invention, répondent à la formule générale I et leurs sels correspondants répondent à la formule générale II :

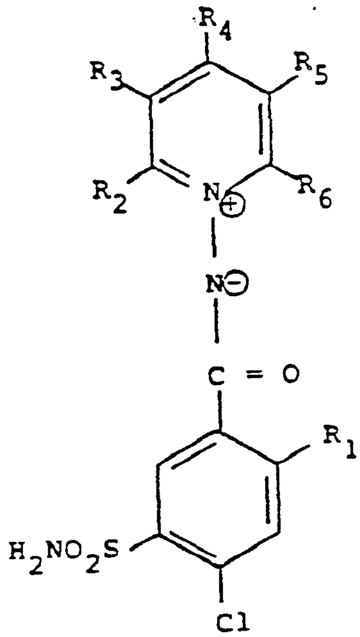

(I)

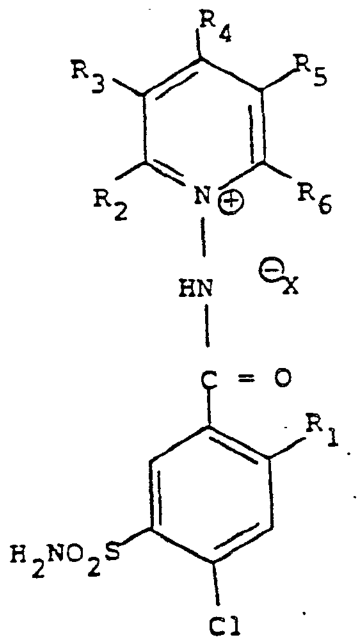

(II)

dans lesquelles :

$R_1$ représente un atome d'hydrogène, un radical 2-furyl-méthylamino ou 2-thénylamino, et

$R_2$, $R_3$, $R_4$, $R_5$ et $R_6$ sont identiques ou différents et représentent un atome d'hydrogène, un radical alkyle inférieur linéaire ou ramifié en $C_1$ à $C_4$, le radical phényle, un radical carbamoyle ou un radical amide ;

$X^{\ominus}$ représente l'anion d'un acide pharmaceutiquement acceptable.

Parmi les anions d'acides pharmaceutiquement acceptables on citera, à titre d'exemples principaux, ceux correspondant aux acides minéraux tels que chlorure, bromure, iodure, nitrate, sulfate, phosphate, ou bien ceux correspondant aux acides organiques tels que acétate, citrate, oxalate, lactate, tartrate, méthansulfonate, benzènesulfonate, p-toluènesulfonate et cyclohexylsulfamate.

La présente invention se rapporte également à la préparation des dérivés de formules générales I et II. Selon l'invention on obtient indifféremment ces dérivés de formule générale I par la mise en œuvre de procédés correspondant aux schémas réactionnels A, B et C, et les sels correspondants de formule générale II par la mise en œuvre du procédé correspondant au schéma réactionnel D.

(Voir Schéma, p. 3)

Schéma réactionnel A

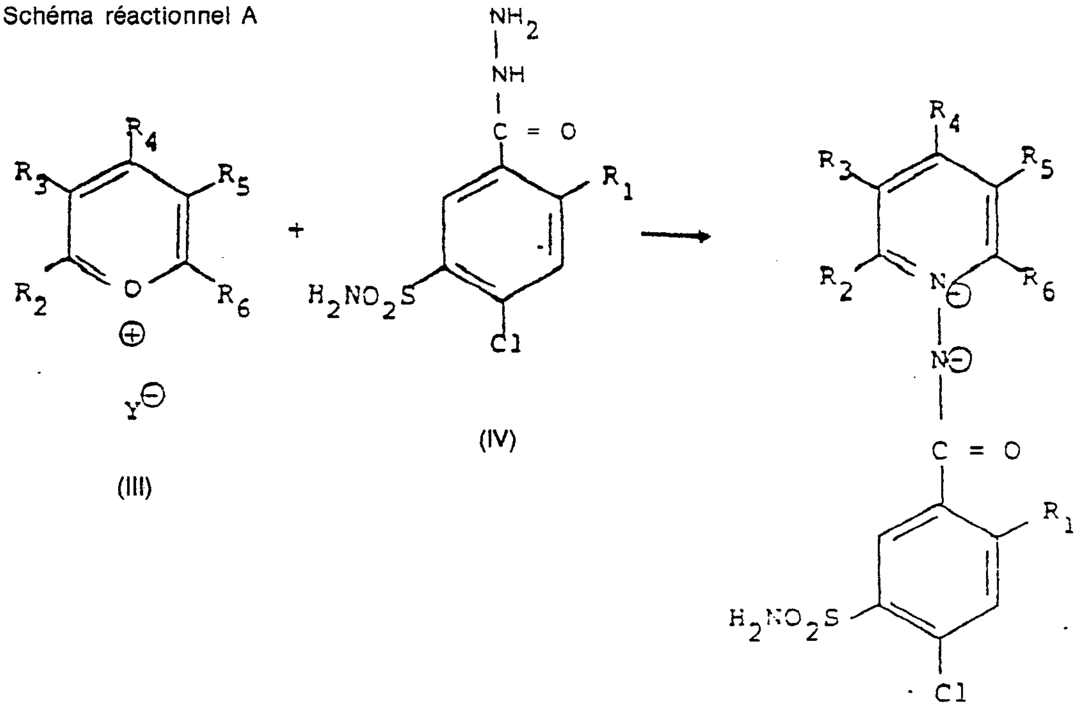

(III) (IV) (I)

Par réaction d'un sel de pyrilium (III), synthétisé par diverses méthodes de préparation en soi connues, et d'une hydrazine monosubstituée (IV), on obtient le sel correspondant lequel traité avec une base, donne lieu à la formation de la bétaïne de formule générale I.

Dans les formules générales III et IV ci-dessus, $R_1$, $R_2$, $R_3$, $R_4$, $R_5$ et $R_6$ ont les significations mentionnées précédemment, et $Y^{\ominus}$ représente les anions $BF_4^{\ominus}$, $ClO_4^{\ominus}$ et $CF_3SO_3^{\ominus}$.

Les sels de pyrilium de formule III peuvent par exemple être obtenus par des méthodes de préparation décrites dans A.T. Balaban & C.D. Nenitzescu, J. Chem. Soc., 1961, 3553-66 ; P.F.G. Praill & A.L. Whitear, J. Chem. Soc., 1961, 3573-9 ; A.T. Balaban, Advanc. Heterocycl. Chem., 1969, 10, 241 ; A.T. Balaban & al., Org. Synt. Coll. Vol. V, 1106-1116 ; K. Dimroth & al., Org. Synt. Coll. Vol. V, 1135 ; A.G. Anderson & P.J. Stang, Org. Synt., 1981, 60, 34 ; E. Elshafie & al., Indian J. Chem. Sect. B, 1981, 20 B (5), 427 ; J.A. Van Allan & G.A. Reynolds, J. Org. Chem., 1968, 33, 1102.

Les détails concernant la mise en œuvre des procédés selon le schéma réactionnel A sont donnés à titre d'illustration dans les exemples.

Schéma réactionnel B

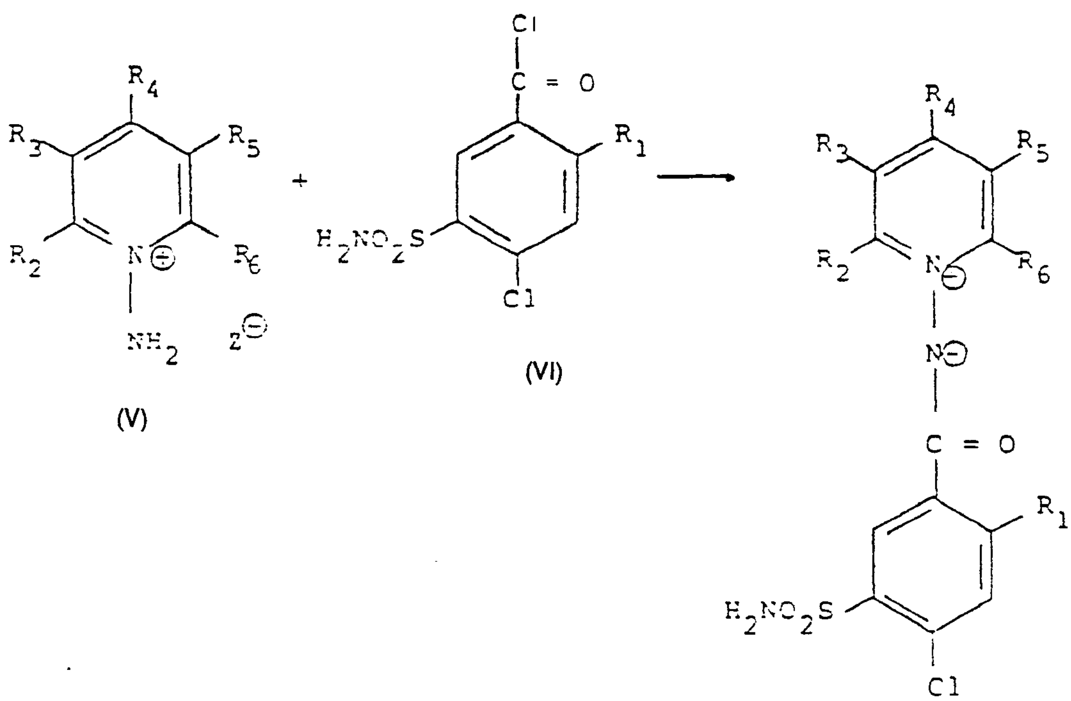

(V) (VI) (I)

Les sels de N-aminopyridinium (V) s'obtiennent par exemple suivant la méthode de R. Gösl & A. Meuwsen, Org. Synt., 1963, 43, 1, en utilisant des solutions aqueuses des pyridines correspondantes et de l'acide hydroxylamino-O-sulfonique ; ou bien suivant la méthode de Y. Tamura, J. Minamikawa, M. Ikeda, Synthesis, 1977, 1, par réaction des pyridines correspondantes avec l'agent d'amination, le O-mésitylensulfonylhydroxylamine.

Par réaction des sels de N-aminopyridinium (V) avec un chlorure d'acide (VI), en milieu basique, on obtient les bétaïnes correspondantes de formule générale I.

Dans les formules générales V et VI, $R_1$, $R_2$, $R_3$, $R_4$, $R_5$ et $R_6$ ont les significations indiquées précédemment et $Z^{\ominus}$ représente les anions :

$Cl^{\ominus}$, $I^{\ominus}$ et 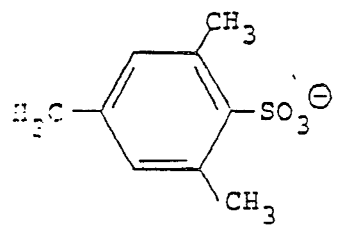

Les détails concernant la mise en œuvre des procédés selon le schéma réactionnel B sont donnés à titre d'illustration dans les exemples.

Schéma réactionnel C

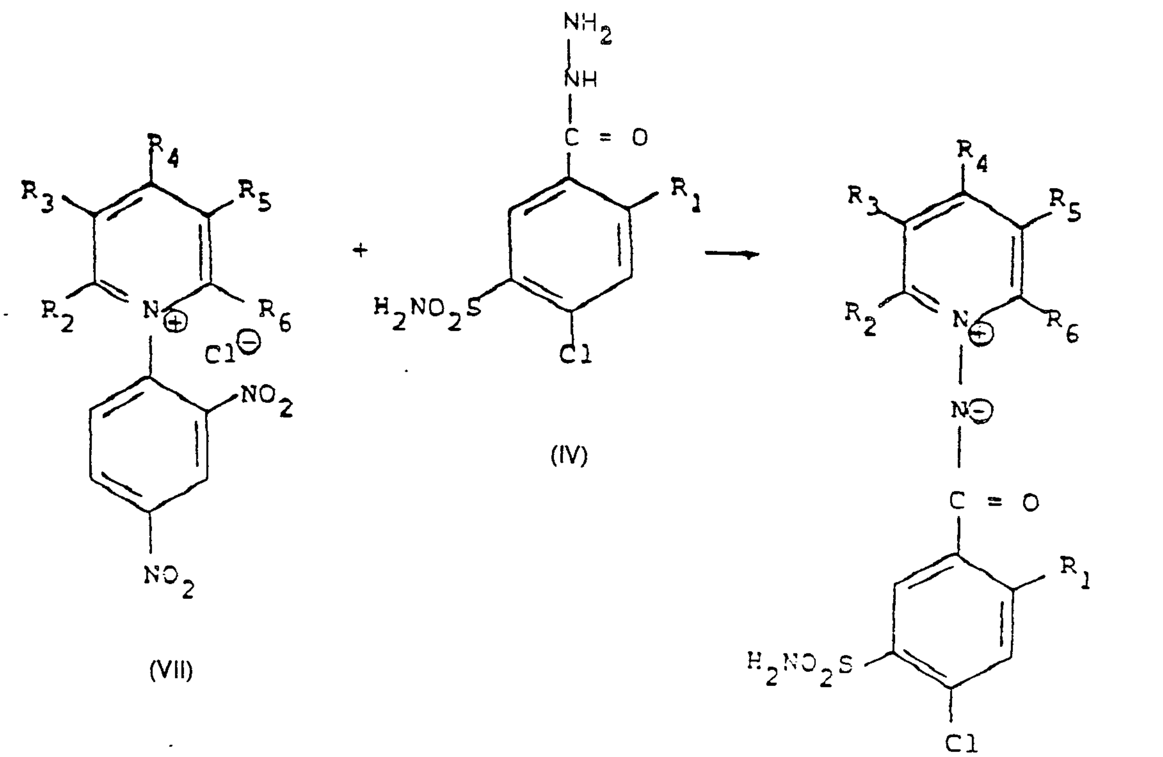

(VII) (IV) (I)

Les chlorures de N-(2,4-dinitrophényl) pyridinium (« Sels de Zincke ») de formule (VII) s'obtiennent par exemple par réaction de 1-chloro-2,4-dinitrobenzène avec la pyridine correspondante (voir T. Zincke, G. Henser & W. Möller, Ann. 1904, 333, 296).

Par réaction du chlorure de N-(2,4-dinitrophényl) pyridinium VII avec une hydrazine monosubstituée IV et une amine tertiaire comme capteur de protons, on obtient les bétaïnes correspondantes de formule générale I.

Dans les formules générales VII et IV ci-dessus, $R_1$, $R_2$, $R_3$, $R_4$, $R_5$ et $R_6$ ont les significations indiquées précédemment.

Les détails concernant la mise en œuvre des procédés selon le schéma réactionnel C sont donnés à titre d'illustration dans les exemples.

Schéma réactionnel D

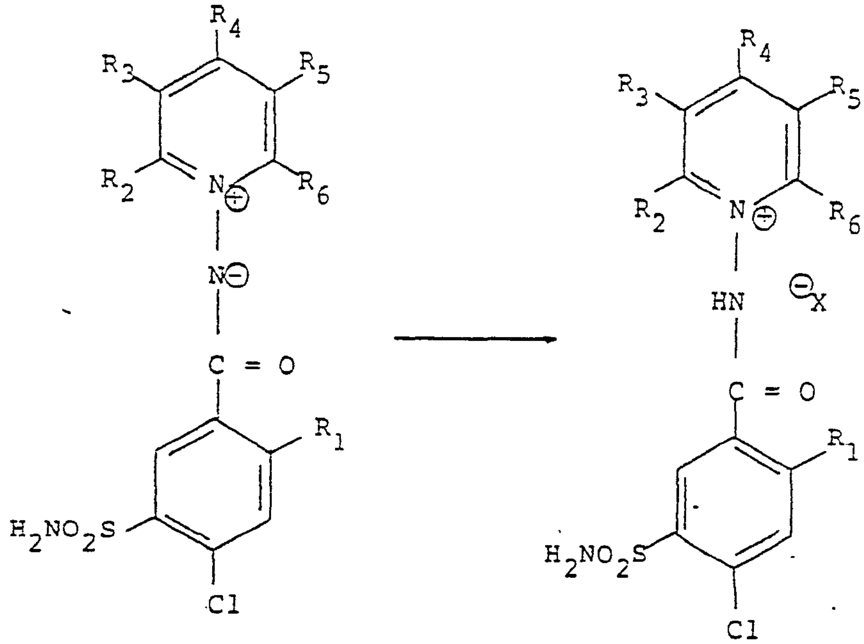

(I) (II)

Par réaction de la bétaïne I avec un acide, $H^{\oplus} X^{\ominus}$, au sein d'un solvant approprié tel qu'un alcool ou une cétone, on obtient les sels correspondants de N-aminopyridinium de formule générale II.

Dans les formules I et II, $R_1$, $R_2$, $R_3$, $R_4$, $R_5$ et $R_6$ et $X^{\ominus}$ ont les significations indiquées précédemment.

Les détails concernant la mise en œuvre des procédés selon le schéma réactionnel D sont donnés à titre d'illustration dans les exemples.

Compte tenu de leur bonne activité diurétique et de leur très faible toxicité, les dérivés de formules générales I et II sont utiles en tant que médicaments administrables en thérapeutique humaine ou animale.

La présente invention se rapporte donc également à l'application de ces dérivés en tant que médicaments, ainsi qu'aux compositions pharmaceutiques les contenant à titre de principe actif.

On décrira ci-après, à titre de simple exemple non limitatif, la préparation de quelques dérivés de formules générales I et II.

## Exemple 1

Préparation de 1-[(4-chloro-3-sulfamoylbenzoyl)amino] pyridinium hydroxyde, sel interne

On met à reflux pendant deux heures une solution de chlorure de N-(2,4-dinitrophényl) pyridinium (8,4 g, 0,03 mol), de 4-chloro-3-sulfamoylbenzoylhydrazine (7,5 g, 0,03 mol) et de triéthylamine (3,2 g, 0,032 mol) dans de l'éthanol (250 ml).

On laisse refroidir, on filtre et on lave successivement avec de l'éthanol, de l'eau, de l'éthanol et finalement de l'éther éthylique. On met à reflux avec 250 ml de dioxane/eau (4 : 1) le précipité ainsi obtenu pendant 24 heures et ensuite on évapore le dioxane sous pression réduite. On traite le résidu avec de l'acide chlorhydrique dilué et on filtre. On neutralise la solution aqueuse avec de l'hydroxyde de sodium en maintenant l'agitation pendant deux heures. On laisse refroidir et on obtient un précipité qui, une fois recristallisé dans l'éthanol aqueux, donne 6,2 g (66 %) du sel interne de l'hydroxyde de 1-[(4-chloro-3-sulfamoylbenzoyl)amino] pyridinium de formule :

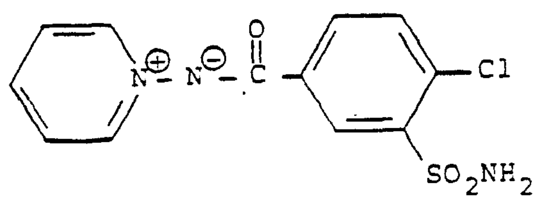

Point de fusion : 225-226 °C
Données spectroscopiques :
IR (KBr) : 1 625, 1 600, 1 550, 1 355, 1 335, 1 160 $cm^{-1}$
$^1H$ RMN, δ, [DMSO ($d_6$)] : 7,25-8,30 (m, 7H) ; 8,60-8,95 (m, 3H).

Exemple 2

Préparation de 1-[(4-chloro-3-sulfamoylbenzoyl)amino]-2,4,6-triméthylpyridinium hydroxyde, sel interne

On met à reflux pendant 4 heures une solution de tétrafluoroborate de triméthylpyrilium (3,87 g, 0,018 4 mol), récemment préparée et de 4-chloro-3-sulfamoylbenzoylhydrazide (5,06 g, 0,020 3 mol) dans l'éthanol (60 ml). On refroidit sous agitation jusqu'à température ambiante et on ajoute de l'hydroxyde de potassium à 85 % (1,22 g, 0,018 5 mol). On agite pendant une heure à température ambiante, on chauffe à ébullition, on filtre à chaud le tétrafluoroborate de potassium formé et on lave le précipité avec de l'éthanol à 60 °C. On obtient par cristallisation de la solution alcoolique concentrée 4,93 g (76 %) de sel interne de l'hydroxyde de 1-[4-chloro-3-sulfamoylbenzoyl)) amino]-2,4,6-triméthylpyridinium de formule :

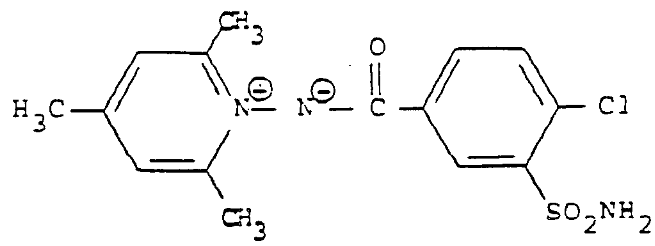

Point de fusion : 264-265 °C
Données spectroscopiques :
IR (KBr) : 1 640, 1 595, 1 545, 1 360, 1 335, 1 165 $cm^{-1}$
$^1H$ RMN, δ, [DMSO ($d_6$)] : 2,5 (s, 9H) ; 3,5 (é, 2H) ;
7,55 (s, 2H) ; 7,60 (d, 1H) ; 8,15 (q, 1H) ; 8,65 (d, 1H).

Exemple 3

Préparation de 1-[(4-chloro-3-sulfamoylbenzoyl)amino]-2,6-diméthylpyridinium hydroxyde, sel interne

On traite l'iodure de 1-amino-2,6-diméthylpyridinium (2,5 g, 0,01 mol) dissous dans l'eau (20 ml) et l'acétone (10 ml), avec du carbonate de potassium (2,8 g, 0,02 ml). Pendant 15 minutes on additionne du chlorure de 4-chloro-3-sulfamoylbenzoyle (2,54 g, 0,01 mol) dissous dans de l'acétone (10 ml). On agite pendant 3 heures à température ambiante et on concentre par évaporation l'acétone à une température inférieure à 40 °C. On extrait avec du chloroforme, on sèche ($Na_2SO_4$), on filtre et on évapore le solvant. Par recristallisation dans l'éthanol du résidu obtenu, on obtient 2,1 g (62 %) du sel interne de l'hydroxyde de 1-[(4-chloro-3-sulfamoybenzoyl)amino]-2,6-diméthylpyridinium, de formule :

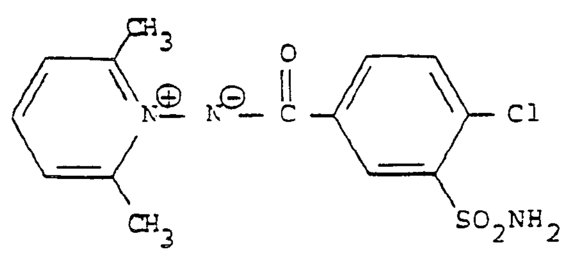

Point de fusion : 266-267 °C
Données spectroscopiques :
IR (KBr) : 1 632, 1 590, 1 540, 1 360, 1 330, 1 165 $cm^{-1}$
$^1H$ RMN, δ, [DMSO ($d_6$)] : 2,47 (s, 6H) ; 7,25-8,50 (m, 8H).

Exemple 4

Préparation de 1-[(4-chloro-3-sulfamoylbenzoyl)amino]-3-carbamoylpyridinium hydroxyde, sel interne

On agite pendant 6 heures à température ambiante une solution de chlorure de 3-carbamoyl-1-(2,4-dinitrophényl) pyridinium (6,5 g, 0,02 mol), de 4-chloro-3-sulfamoylbenzoylhydrazide (5 g, 0,02 mol) et de triéthylamine (2,15 g, 0,021 mol) dans le méthanol (50 ml). On filtre et on lave avec du méthanol et finalement de l'éther éthylique. On met à reflux pendant 15 heures le solide obtenu en suspension dans le dioxane/$H_2O$ (4 : 1) (100 ml), puis on évapore le dioxane sous pression réduite. On acidifie le résidu avec de l'acide chlorhydrique dilué, on filtre et on lave à l'eau. On agite le précipité obtenu avec de l'éthanol à ébullition et on filtre à chaud. On agite le précipité avec une solution diluée d'hydroxyde de sodium pendant une heure, on filtre et on lave avec de l'eau. On obtient 4,1 g (57 %) du sel interne d'hydroxyde de 1-[(4-chloro-3-sulfamoylbenzoyl) amino]-3-carbamoylpyridinium de formule :

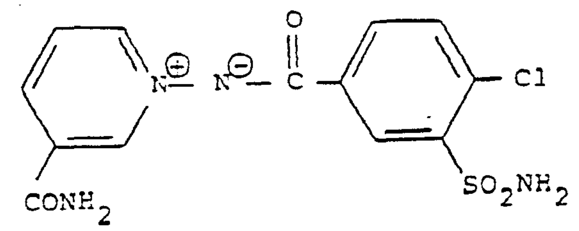

Point de fusion : 273-274 °C
Données spectroscopiques :
IR (KBr) : 1 700, 1 638, 1 595, 1 545, 1 355, 1 335, 1 175 $cm^{-1}$
$^1H$ RMN, δ, [DMSO] ($d_6$)] : 7,5-8,75 (m, 9H) ; 8,9 (d, 1H) ; 9,18 (s, 1H).

Exemple 5

Préparation de 1-{[4-chloro-2-(2-furylméthylamino)-5-sulfamoylbenzoyl]amino}-2,4,6-triméthylpyridinium hydroxyde, sel interne

On met à reflux pendant six heures une solution de tétrafluoroborate de triméthylpyrilium (3,87 g, 0,018 4 mol) récemment préparée et du 4-chloro-2-(2-furylméthylamino)-5-sulfamoylbenzoylhydrazide (7,21 g, 0,02 mol) dans 70 ml d'éthanol. On refroidit à température ambiante avec agitation, on ajoute de l'hydroxyde de potassium à 85 % (1,22 g, 0,018 5 mol) et on maintient l'agitation pendant une heure. On filtre et on extrait à plusieurs reprises avec de l'éthanol. On concentre la solution alcoolique et on obtient 4,7 g (55 %) du sel interne de l'hydroxyde de 1-{[4-chloro-2-(2-furylméthylamino)-5-sulfamoylbenzoyl]amino}-2,4,6-triméthylpyridinium, de formule :

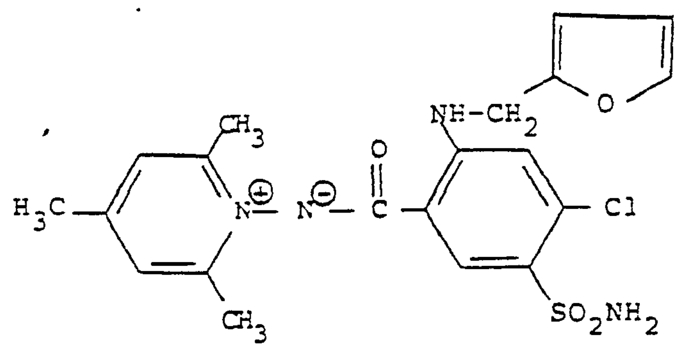

Point de fusion : 274-275 °C
Données spectroscopiques :
IR (KBr) : 1 638, 1 600, 1 560, 1 355, 1 340, 1 260, 1 165 $cm^{-1}$
$^1H$ RMN, δ, [DMSO ($d_6$)] : 2,5 (s, 9H) ; 4,51 (s, 2H) ; 6,35 (d, 2H) ; 6,92 (s, 1H) ; 7,18 (s, 2H) ; 7,62 (s, 3H) ; 8,63 (s, 1H) ; 9,5 (é, 1H).

Exemple 6

Préparation de 1-[(4-chloro-3-sulfamoylbenzoyl)amino]-2,4,6-triphénylpyridinium hydroxyde, sel interne

On met à reflux pendant quinze heures une solution de tétrafluoroborate de triphénylpyrilium (3,96 g, 0,01 mol) et de 4-chloro-3-sulfamoylbenzoylhydrazide (2,97 g, 0,011 mol) dans l'éthanol (50 ml). On refroidit avec agitation à température ambiante et on ajoute de l'hydroxyde de potassium à 85 % (0,68 g, 0,010 3 mol). On maintient pendant quinze minutes sous agitation et on filtre. On évapore à sec le filtrat, on recristallise dans le benzène et on obtient 3,0 g (52 %) du sel interne de l'hydroxyde de 1-[(4-chloro-3-sulfamoylbenzoyl)amino]-2,4,6-triphénylpyridinium, de formule :

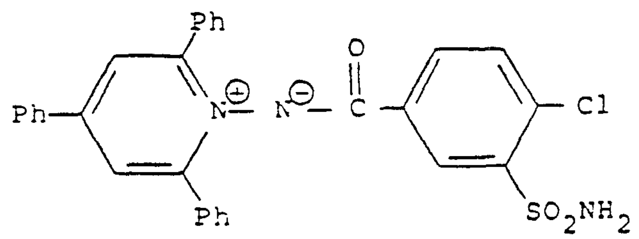

Point de fusion : 170-172 °C
Données spectroscopiques :
IR (KBr) : 1 628, 1 600, 1 550, 1 350, 1 340, 1 165 $cm^{-1}$
$^{1}H$ RMN, δ, [DMSO ($d_6$)] : 7,2-7,85 (m, 17H) ; 7,92-8,25 (m, 5H).

## Exemple 7

Préparation de 1-[(4-chloro-3-sulfamoylbenzoyl)amino]-2,4,6-triméthylpyridinium, chlorure

On ajoute, sous agitation, 10 ml d'éthanol saturé d'acide chlorhydrique à une solution du sel interne de l'hydroxyde de 1-[(4-chloro-3-sulfamoylbenzoyl)amino]-2,4,6-triméthylpyridinium (3,5 g, 0,01 mol) dans l'éthanol (80 ml). Après une heure d'agitation on filtre le précipité formé et on lave à l'éthanol. On obtient 3,6 g (92 %) du chlorure de 1-[(4-chloro-3-sulfamoylbenzoyl)amino]-2,4,6-triméthylpyridinium, de formule :

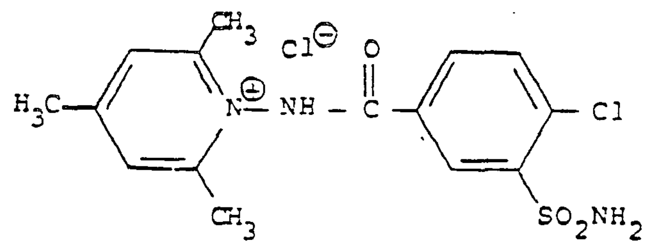

Point de fusion : 272-274 °C
Données spectroscopiques :
IR (KBr) : 1 705, 1 642, 1 348, 1 165 $cm^{-1}$
$^{1}H$ RMN, δ, [DMSO ($d_6$)] : 2,6 (s, 3H) ; 2,7 (s, 6H) ; 4,85 (é, 2H) ; 7,7-8,05 (m, 4H) ; 8,35-8,65 (m, 2H).

## Exemple 8

Préparation de 1-{[4-chloro-2-(2-furylméthylamino)-5-sulfamoylbenzoyl]amino}-2,4,6-triméthylpyridinium chlorure, chlorhydrate

On ajoute, sous agitation, 20 ml d'éthanol saturé d'acide chlorhydrique à une suspension du sel interne de l'hydroxyde de 1-{(4-chloro-2-(2-furylméthylamino)-5-sulfamoylbenzoyl]amino}-2,4,6-triméthyl-pyridinium (4,6 g, 0,01 mol) dans l'éthanol (40 ml). Au bout de quelques secondes on obtient une solution transparente qui commence à précipiter immédiatement. On filtre, on lave à l'éthanol et on obtient 4,9 g (98 %) de chlorhydrate du chlorure de 1-{[4-chloro-2-(2-furylméthylamino)-5-sulfamoylbenzoyl]amino}-2,4,6-triméthylpyridinium, de formule :

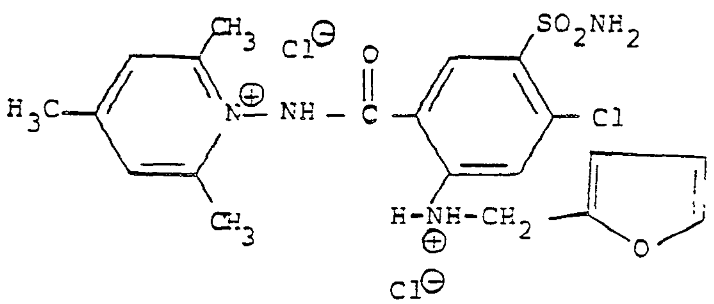

Point de fusion : 257-258 °C
Données spectroscopiques :
IR (KBr) : 1 668, 1 635, 1 565, 1 355, 1 165 $cm^{-1}$
$^{1}H$ RMN, δ, [DMSO ($d_6$)] : 2,6 (s, 3H) ; 2,7 (s, 6H) ; 4,6 (s, 2H) ; 5-6 (é, 5H) ; 6,4 (d, 2H) , 7,1 (s, 1H) ; 7,6 (s, 1H) ; 7,95 (s, 2H) , 8,73 (s, 1H).

Exemple 9

Préparation de 1-[(4-chloro-3-sulfamoylbenzoyl)amino]-2,4,6-triphénylpyridinium, chlorure

On ajoute, sous agitation, 10 ml d'éthanol saturé d'acide chlorhydrique à une solution du sel interne de l'hydroxyde de 1-[(4-chloro-3-sulfamoylbenzoyl)amino]-2,4,6-triphénylpyridinium (5,4 g, 0,01 mol) dans l'éthanol (25 ml). Après une demi-heure d'agitation on filtre le précipité formé et on obtient 5,2 g (90 %) de chlorure de 1-[(4-chloro-3-sulfamoylbenzoyl)amino]-2,4,6-triphénylpyridinium, de formule :

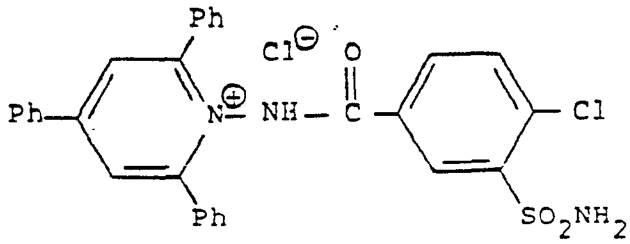

Point de fusion : 290-292 °C
Données spectroscopiques :
IR (KBr) : 1 700, 1 628, 1 340, 1 170 $cm^{-1}$
$^1H$ RMN, δ, [DMSO ($d_6$)] : 4,5 (é, 3H) ; 7,25-7,73 (m, 11H) ; 7,73-8,3 (m, 7H) ; 8,53 (s, 2H).

Activité diurétique
Méthode de R.M. Taylor, J.G. Topliss, J. Med. Pharm. Chem. 1962, 4, 312

On utilise des rats mâles Sprague-Dawley (HC/CFY) d'un poids compris entre 150 et 200 grammes. On supprime aux animaux l'alimentation et la boisson 16 heures avant le début de l'essai. Les produits sont administrés en suspension dans une suspension de carboxyméthylcellulose à 0,5 % dans une solution de chlorure de sodium à 0,9 % moyennant une sonde œsophagique à raison de 50 ml/kg de poids corporel. On place les animaux dans des cages de métabolisme individuelles et on recueille la totalité de l'urine excrétée après les intervalles de temps suivants : 1, 2, 3, 4, 6 et 8 heures.

Dans l'urine on détermine les paramètres suivants : volume (ml/kg) ; sodium et potassium (mEqV/kg/8 h) (photomètre de flamme) ; chlorure (mEqV/kg/8 h) (chlorurmètre) ; pH/8 heures (pHmètre) et pression osmotique (mOsmol/kg/8 h) (osmomètre).

Les animaux témoins reçoivent la suspension de carboxyméthylcellulose à 0,5 % dans une solution de chlorure de sodium à 0,9 % à raison de 50 ml/kg de poids corporel.

Moyennant le test statistique de la « t » de Student pour des valeurs indépendantes, on compare les valeurs des paramètres mentionnés antérieurement des lots traités à la dose de 40 mg/kg et du lot témoin. On considère qu'un produit présente une activité diurétique lorsque la différence entre le lot témoin et le lot traité est significative (P < 0,05) (Blin, C.I., 1970, « Statistics in Biology, vol. II, Mc Graw-Hill, New York).

Dans le tableau 1 ci-après on indique les volumes d'urine excrétée à différents intervalles de temps après administration de 40 mg/kg de divers composés selon l'invention.

(Voir Tableau 1 p. 10)

Tableau 1

| Produit | Elimination urinaire (ml/kg) | | | | | |
|---|---|---|---|---|---|---|
| | 1 h | 2 h | 3 h | 4 h | 6 h | 8 h |
| Témoin | 4,4 | 12,3 | 17,2 | 21,3 | 25,0 | 27,6 |
| Exemple 2 | 10,4 *** | 22,3 *** | 30,2 *** | 36,0 *** | 41,5 *** | 45,6 *** |
| Exemple 1 | 7,3 * | 15,2 N.S | 27,6 *** | 35,4 *** | 40,5 *** | 44,5 *** |
| Exemple 4 | 5,8 N.S | 8,6 N.S | 19,5 N.S | 24,2 N.S | 31,1 N.S | 37,5 * |
| Exemple 7 | 6,2 N.S | 18,6 ** | 28,4 *** | 36,1 *** | 47,3 *** | 52,4 *** |
| Exemple 5 | 1,1 N.S | 3,4 ** | 8,0 * | 12,5 * | 18,4 N.S | 20,7 N.S |
| Exemple 8 | 0,0 N.S | 11,9 N.S | 19,6 N.S | 28,5 N.S | 34,4 * | 39,2 ** |
| Exemple 3 | 8,4 N.S | 22,9 ** | 31,9 ** | 36,7 *** | 45,2 *** | 50,7 *** |
| Exemple 6 | 6,9 N.S | 14,2 N.S | 19,4 N.S | 22,2 N.S | 26,0 N.S | 27,4 N.S |
| Exemple 9 | 5,2 N.S | 12,6 N.S | 16,8 N.S | 19,0 N.S | 21,5 N.S | 25,3 N.S |
| Chlortalidone | 8,7 *** | 21,0 *** | 30,9 *** | 37,6 *** | 45,6 *** | 49,8 *** |

N.S. : Non significatif (P > 0,05) ; * : significatif (P < 0,05) ; ** : très significatif (P < 0,01) ; *** : hautement significatif (P < 0,001).

Dans le tableau 2 ci-après on indique les différents paramètres (Na, K, Cl, pH, pression osmotique) mesurés dans l'urine totale excrétée pendant 8 heures, après administration de 40 mg/kg de divers composés selon l'invention.

Tableau 2

| Produit | Elimination urinaire (8 heures) | | | | |
|---|---|---|---|---|---|
| | Na | K | Cl | pH | Pression osmotique |
| Témoin | 5,6 | 1,2 | 6,4 | 6,1 | 20,7 |
| Exemple 2 | 9,1 *** | 1,7 *** | 11,8 *** | 6,0 N.S | 29,7 *** |
| Exemple 1 | 8,3 *** | 1,5 ** | 9,2 *** | 6,6 * | 25,5 *** |
| Exemple 4 | 6,6 N.S | 1,1 N.S | 7,0 N.S | 6,2 N.S | 20,8 N.S |
| Exemple 7 | 8,9 *** | 1,6 ** | 9,3 *** | 6,3 N.S | 31,0 *** |
| Exemple 5 | 3,0 *** | 1,3 N.S | 6,2 N.S | 6,1 N.S | 16,6 N.S |
| Exemple 8 | 5,9 N.S | 1,2 N.S | 7,2 N.S | 6,7 ** | 23,4 N.S |
| Exemple 3 | 9,5 *** | 1,8 *** | 11,7 *** | 6,1 N.S | 34,2 *** |
| Exemple 6 | 6,6 * | 1,1 N.S | 8,1 ** | 6,0 N.S | 25,3 ** |
| Exemple 9 | 6,0 N.S | 1,2 N.S | 7,5 * | 6,2 N.S | 25,1 ** |
| Chlortalidone | 9,3 *** | 1,9 *** | 10,8 *** | 6,6 * | 29,9 *** |

A titre d'exemple non limitatif, dans le tableau 3 on mentionne les doses efficaces cinquante correspondant au volume d'urine et à la pression osmotique des composés des exemples 1, 2 et 3 comparés à la chlortalidone.

Tableau 3

| Produit | $DE_{50}$ (mg/kg) [1] | |
|---|---|---|
| | Volume d'urine (ml/kg/8 h) | Pression osmotique (m osmol/kg/8 h) |
| Exemple 1 | 4,1 | 1,9 |
| Exemple 2 | 4,7 | 1,2 |
| Exemple 3 | 4,6 | 2,1 |
| Chlortalidone | 6,9 | 7,7 |

[1] La $DE_{50}$ a été calculée à partir de la droite de régression de la représentation du logarithme décimal de la dose face au pourcentage d'effet obtenu.

Toxicité aiguë

On administre le produit par voie orale, en suspension dans la gomme arabique à 5 % des souris albinos CFLP-RE de 20-25 g et à des rats Sprague-Dawley CFY-RE de 125-175 g. Le volume administré est de 25 ml/kg chez la souris sauf pour la dose maximale de 12 800 mg/kg où l'on a administré un volume de 50 ml/kg, et de 10 ml/kg chez le rat sauf pour la dose maximale de 12 800 mg/kg où l'on a administré un volume de 30 ml/kg.

Il a été impossible de déterminer la dose léthale 50 étant donné l'absence de mortalité.

Les résultats obtenus pour l'exemple 2 sont mentionnés à titre d'illustration dans le tableau 4.

Tableau 4

| Dose mg / kg | Mortalité | | | |
|---|---|---|---|---|
| | Souris | | Rat | |
| | ♂ | ♀ | ♂ | ♀ |
| 400 | 0/4 | 0/4 | - | - |
| 800 | 0/4 | 0/4 | - | - |
| 1600 | 0/4 | 0/4 | 0/4 | 0/4 |
| 3200 | 0/4 | 0/4 | 0/4 | 0/4 |
| 6400 | 0/8 | 0/8 | 0/4 | 0/4 |
| 12800 | 0/8 | 0/8 | 0/8 | 0/8 |

En thérapeutique humaine, la dose proposée des dérivés de la présente invention est environ comprise entre 20 et 60 mg/jour, par exemple administrée sous forme de comprimés.

On indiquera ci-après à titre d'exemple, une forme gallénique particulière des dérivés de la présente invention.

Exemple de formule par comprimé

| | |
|---|---|
| 1-[(4-chloro-3-sulfamoylbenzoyl) amino] 2,4,6-triméthylpyridinium hydroxyde, sel interne | 0,020 g |
| Lactose | 0,101 5 g |
| Amidon | 0,027 g |
| Cellulose microcristalline | 0,018 g |
| Amidon prégélatinisé | 0,005 4 g |
| Polivinylpyrrolidone | 0,005 4 g |
| Stéarate de magnésium | 0,001 8 g |
| Dioxyde de silice colloïdal | 0,000 9 g |
| | 0,180 0 g |

Les compositions pharmaceutiques conformes à la présente invention, du fait de leur propriété salidiurétique, peuvent être utilisées efficacement pour le traitement des œdèmes cardiaques, rénaux et hépatiques, pour le traitement de l'insuffisance cardiaque et de l'hypertension artérielle et pour assurer le blocage de la lactation.

Il est bien entendu que les compositions pharmaceutiques conformes à la présente invention peuvent contenir à titre de principe actif un ou plusieurs dérivés selon l'invention en association avec d'autres principes actifs d'activités différentes ou complémentaires telles que par exemple une substance à activité bêta-bloquante utile dans le traitement de l'hypertension.

**Revendications** (pour les Etats contractants : BE, CH, DE, FR, GB, IT, LI, LU, NL, SE)

1. Dérivés de sels internes d'hydroxyde d'aminopyridinium et leurs sels correspondants répondant respectivement aux formules générales I et II :

(Voir Schéma p. 13)

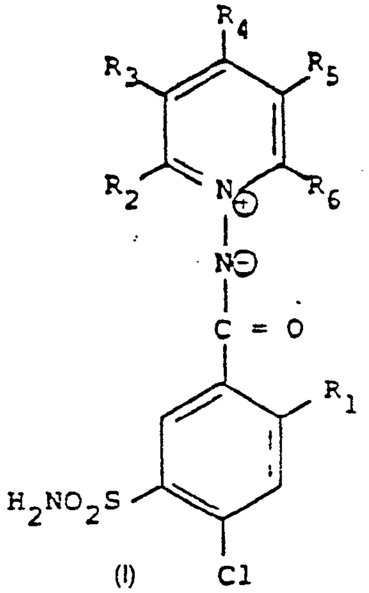

(I)

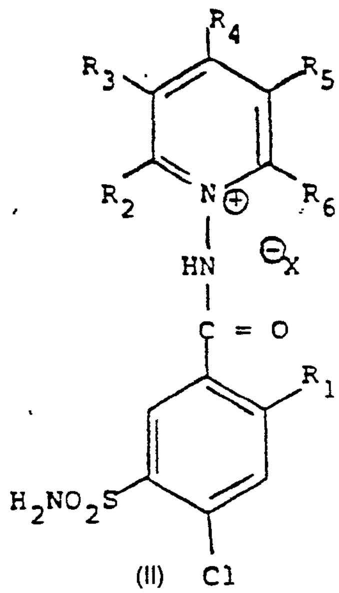

(II)

dans lesquelles :

$R_1$ représente un atome d'hydrogène, un radical 2-furyl-méthylamino ou 2-thénylamino, et

$R_2$, $R_3$, $R_4$, $R_5$ et $R_6$ sont identiques ou différents et représentent un atome d'hydrogène, un radical alkyle inférieur linéaire ou ramifié en $C_1$ à $C_4$, le radical phényle, un radical carbamoyle ou un radical amide ;

$X^{\ominus}$ représente l'anion d'un acide pharmaceutique acceptable.

2. Un dérivé de formule générale I selon la revendication 1, choisi parmi :

le sel interne de l'hydroxyde de 1-[(4-chloro-3-sulfamoylbenzoyl) amino] pyridinium ;

le sel interne de l'hydroxyde de 1-[(4-chloro-3-sulfamoylbenzoyl) amino]-2,4,6-triméthylpyridinium ;

le sel interne de l'hydroxyde de 1-[(4-chloro-3-sulfamoylbenzoyl) amino]-2,6-diméthylpyridinium ;

le sel interne de l'hydroxyde de 1-[(4-chloro-3-sulfamoylbenzoyl) amino]-3-carbamoylpyridinium ;

le sel interne de l'hydroxyde de 1-{[4-chloro-2-(2-furylméthylamino)-5-sulfamoylbenzoyl] amino}-2,4,6-triméthylpyridinium, et

le sel interne de l'hydroxyde de 1-[(4-chloro-3-sulfamoylbenzoyl) amino]-2,4,6-triphénylpyridinium.

3. Un dérivé de formule générale II selon la revendication 1, choisi parmi :

le chlorure de 1-[(4-chloro-3-sulfamoylbenzoyl)-amino]-2,4,6-triméthylpyridinium ;

le chlorure de 1-{[4-chloro-2-(2-furylméthylamino)-5-sulfamoylbenzoyl]-amino}-2,4,6-triméthylpyridinium, et

le chlorure de 1-[(4-chloro-3-sulfamoylbenzoyl) amino]-2,4,6-triphénylpyridinium.

4. Procédé de préparation des dérivés de formule générale I selon la revendication 1, caractérisé en ce que l'on fait réagir un sel de pyrilium de formule III

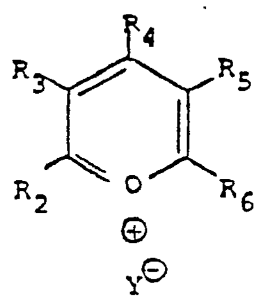

(III)

avec une hydrazine monosubstituée de formule IV

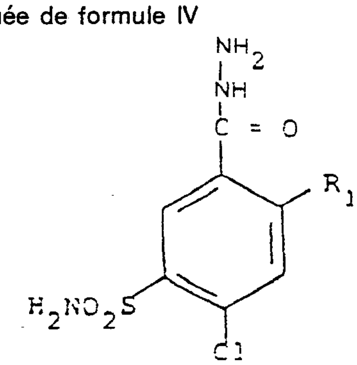

(IV)

dans lesquelles $R_1$, $R_2$, $R_3$, $R_4$, $R_5$ et $R_6$ ont les significations données à la revendication 1, et $Y^{\ominus}$ représente les anions $BF_4^{\ominus}$, $ClO_4^{\ominus}$ et $CF_3SO_3^{\ominus}$, et en ce que le sel correspondant obtenu est traité avec une base pour former la bétaïne de formule générale I.

5. Procédé de préparation des dérivés de formule générale I, selon la revendication 1, caractérisé en ce que l'on fait réagir un sel de N-aminopyridinium de formule générale V

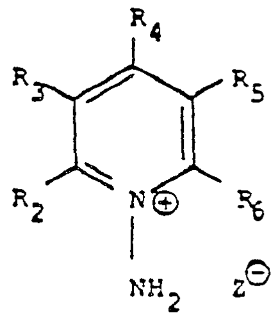

(V)

avec un chlorure d'acide de formule générale VI

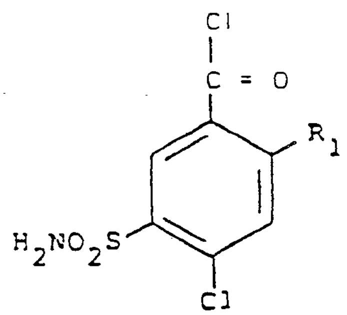

(VI)

dans lesquelles $R_1$, $R_2$, $R_3$, $R_4$, $R_5$ et $R_6$ ont les significations données à la revendication 1, et $Z^{\ominus}$ représente les anions

$Cl^{\ominus}$ , $I^{\ominus}$ et 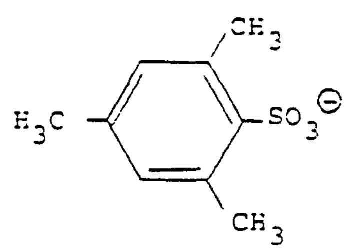

et en ce que l'on obtient en milieu basique la bétaïne correspondante de formule générale I.

6. Procédé de préparation des dérivés de formule générale I, selon la revendication 1, caractérisé en ce que l'on fait réagir le chlorure de N-(2,4-dinitrophényl) pyridinium de formule générale VII

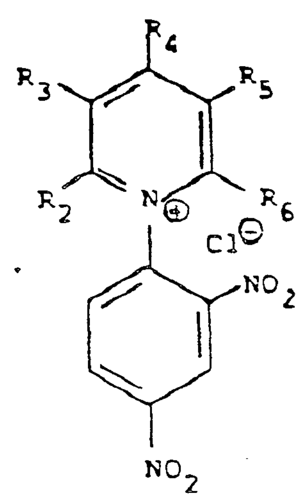

(VII)

avec une hydrazine monosubstituée de formule générale IV

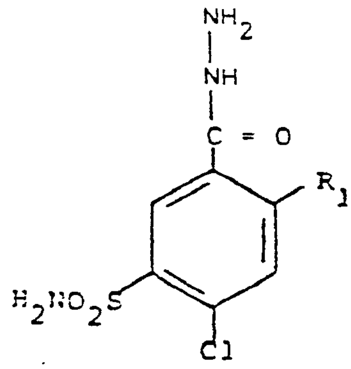

(IV)

dans lesquelles $R_1$, $R_2$, $R_3$, $R_4$, $R_5$ et $R_6$ ont les significations indiquées à la revendication 1, et en ce que la réaction est conduite en présence d'une amine tertiaire utilisée comme capteur de protons, pour obtenir les bétaïnes correspondantes de formule générale I.

7. Procédé de préparation des dérivés de formule générale II selon la revendication 1, caractérisé en ce que l'on fait réagir un dérivé de formule générale I

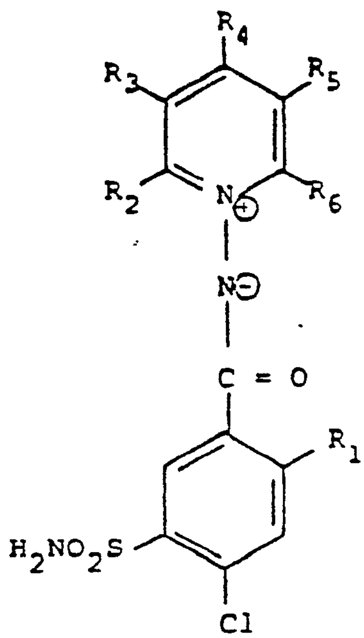

(I)

dans laquelle :

$R_1$ représente un atome d'hydrogène, un radical 2-furyl-méthylamino ou 2-thénylamino, et

$R_2$, $R_3$, $R_4$, $R_5$ et $R_6$ sont identiques ou différents et représentent un atome d'hydrogène, un radical alkyle inférieur linéaire ou ramifié en $C_1$ à $C_4$, le radical phényle, un radical carbamoyle ou un radical amide, avec un acide de formule $H^{\oplus}X^{\ominus}$, au sein d'un solvant approprié tel qu'un alcool ou une cétone, pour obtenir les sels correspondants de N-aminopyridinium de formule générale II.

8. A titre de médicaments nouveaux, les dérivés de formules générales I et II selon l'une des revendications 1, 2 et 3.

9. Composition pharmaceutique, caractérisée en ce qu'elle contient outre un support pharmaceutiquement acceptable, au moins un dérivé de formule générale I ou II selon l'une des revendications 1, 2 et 3.

**Revendications** (pour l'Etat contractant AT)

1. Procédé de préparation des dérivés de sels internes d'hydroxyde d'aminopyridinium répondant à la formule générale

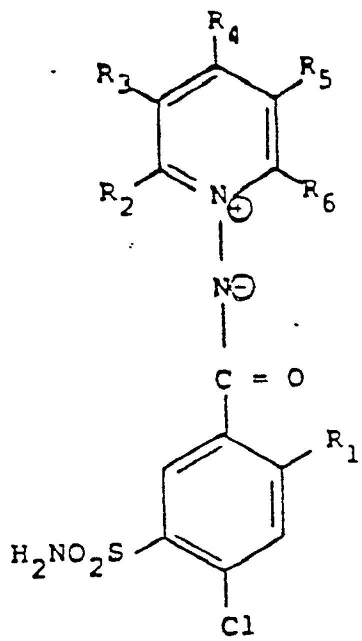

(I)

dans laquelle :

$R_1$ représente un atome d'hydrogène, un radical 2-furyl-méthylamino ou 2-thénylamino, et

$R_2$ à $R_6$ sont identiques ou différents et représentent un atome d'hydrogène, un radical alkyle inférieur linéaire ou ramifié en $C_{1-4}$, le radical phényle, un radical carbamoyle ou un radical amide, caractérisé en ce que l'on fait réagir un sel de pyrilium de formule III :

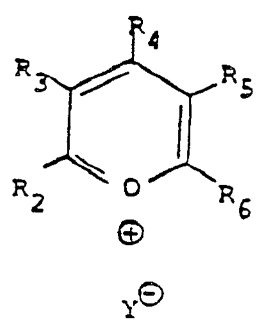

(III)

avec une hydrazine monosubstituée de formule IV

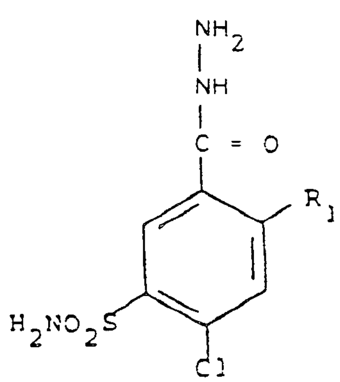

(IV)

dans lesquelles : $R_1$, $R_2$, $R_3$, $R_4$, $R_5$ et $R_6$ ont les significations données ci-dessus et $Y^{\ominus}$ représente les anions $BF_4^{\ominus}$, $ClO_4^{\ominus}$ et $CF_3SO_3^{\ominus}$, et en ce que le sel correspondant obtenu est traité avec une base pour former la bétaïne de formule générale I.

2. Procédé de préparation des dérivés de formule générale I, selon la revendication 1, caractérisé en ce que l'on fait réagir un sel de N-aminopyridinium de formule générale V :

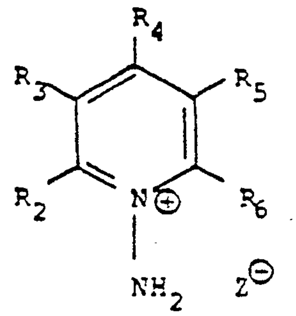

(V)

avec un chlorure d'acide de formule générale VI

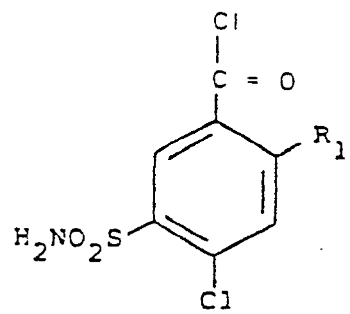

(VI)

dans lesquelles $R_1$, $R_2$, $R_3$, $R_4$, $R_5$ et $R_6$ ont les significations données à la revendication 1, et $Z^{\ominus}$ représente les anions

$Cl^{\ominus}$ , $I^{\ominus}$ et 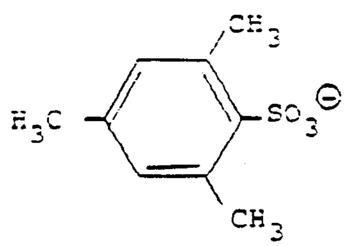

et en ce que l'on obtient en milieu basique la bétaïne correspondante de formule générale I.

3. Procédé de préparation des dérivés de formule générale I, selon la revendication 1, caractérisé en ce que l'on fait réagir le chlorure de N-(2,4-dinitrophényl) pyridinium de formule générale VII

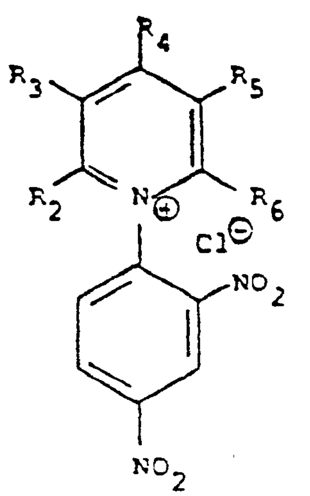

(VII)

avec une hydrazine monosubstituée de formule générale IV

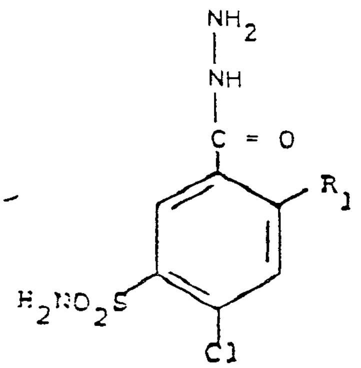

(IV)

dans lesquelles $R_1$, $R_2$, $R_3$, $R_4$, $R_5$ et $R_6$ ont les significations indiquées à la revendication 1, et en ce que la réaction est conduite en présence d'une amine tertiaire utilisée comme capteur de protons, pour obtenir les bétaïnes correspondantes de formule générale I.

4. Procédé de préparation de sels correspondants aux dérivés de sels internes d'hydroxyde d'aminopyridinium répondant à la formule générale II :

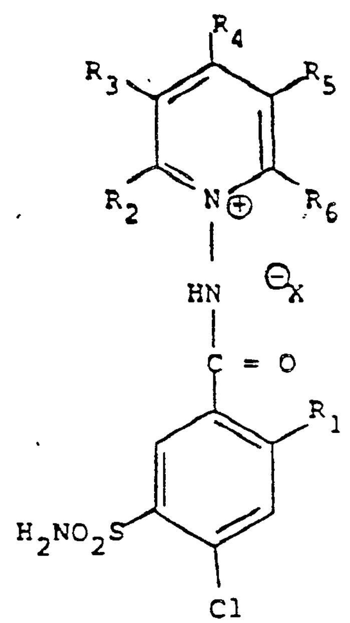

(II)

dans laquelle :

$R_1$ représente un atome d'hydrogène, un radical 2-furyl-méthylamino ou 2-thénylamino, et

$R_2$ à $R_6$ sont identiques ou différents et représentent un atome d'hydrogène, un radical alkyle inférieur linéaire ou ramifié en $C_{1-4}$, le radical phényle, un radical carbamoyle ou un radical amide,

$X^{\ominus}$ représente l'anion d'un acide pharmaceutiquement acceptable,

caractérisé en ce que l'on fait réagir un dérivé de formule générale I, selon la revendication 1, avec un acide de formule $H^{\oplus}X^{\ominus}$, au sein d'un solvant approprié tel qu'un alcool ou une cétone, pour obtenir les sels correspondants de N-aminopyridinium de formule générale II.

5. Procédé de préparation d'un dérivé de formule générale I, selon la revendication 1, caractérisé en ce que l'on prépare un dérivé choisi parmi :

le sel interne de l'hydroxyde de 1-[(4-chloro-3-sulfamoylbenzoyl) amino] pyridinium ;

le sel interne de l'hydroxyde de 1-[(4-chloro-3-sulfamoylbenzoyl) amino]-2,4,6-triméthylpyridinium ;

le sel interne de l'hydroxyde de 1-[(4-chloro-3-sulfamoylbenzoyl) amino]-2,6-diméthylpyridinium ;

le sel interne de l'hydroxyde de 1-[(4-chloro-3-sulfamoylbenzoyl) amino]-3-carbamoylpyridinium ;

le sel interne de l'hydroxyde de 1-{[4-chloro-2-(2-furylméthylamino)-5-sulfamoylbenzoyl] amino}-2,4,6-triméthylpyridinium, et

le sel interne de l'hydroxyde de 1-[(4-chloro-3-sulfamoylbenzoyl) amino]-2,4,6-triphénylpyridinium.

6. Procédé de préparation du sel d'un dérivé de formule générale II, selon la revendication 4, choisi parmi :

le chlorure de 1-[(4-chloro-3-sulfamoylbenzoyl) amino]-2,4,6-triméthylpyridinium ;

le chlorure de 1-{[4-chloro-2-(2-furylméthylamino)-5-sulfamoylbenzoyl] amino}-2,4,6-triméthylpyridinium, et

le chlorure de 1-[(4-chloro-3-sulfamoylbenzoyl) amino]-2,4,6-triphénylpyridinium.

**Claims** (for the Contracting States : BE, CH, DE, FR, GB, IT, LI, LU, NL, SE)

1. Derivatives of internal salts of aminopyridinium hydroxide and their corresponding salts corresponding to the general formulae I and II respectively :

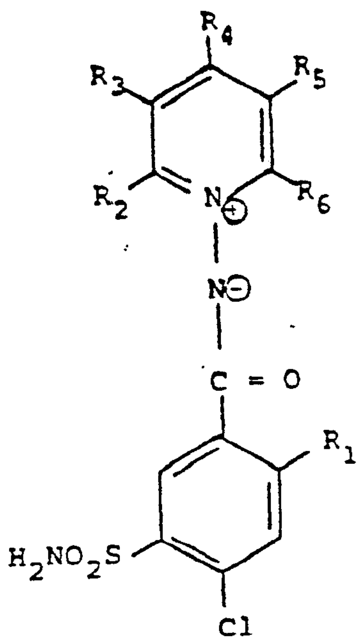

(I)

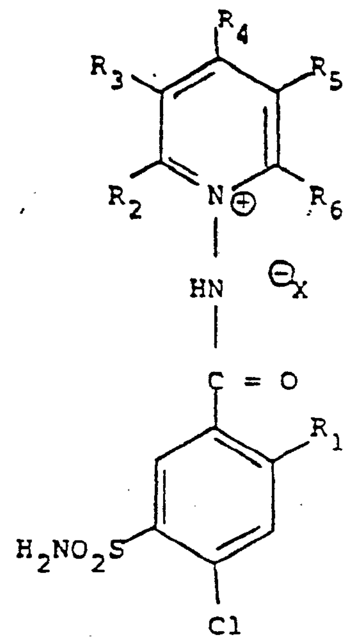

(II)

in which

$R_1$ represents a hydrogen atom, a 2-furyl-methylamino or 2-thenylamino radical and

$R_2$, $R_3$, $R_4$, $R_5$ and $R_6$ are identical or different and represent a hydrogen atom, a $C_1$-$C_4$ linear or branched lower alkyl radical, the phenyl radical, a carbamoyl radical or an amide radical ;

$X^{\ominus}$ represents the anion of a pharmaceutically acceptable acid.

2. A derivative corresponding to general formula I according to Claim 1 selected from

the internal salt of 1-[(4-chloro-3-sulphamoylbenzoyl) amino] pyridinium hydroxide ;

the internal salt of 1-[(4-chloro-3-sulphamoylbenzoyl) amino]-2,4,6-trimethylpyridinium hydroxide ;

the internal salt of 1-[(4-chloro-3-sulphamoylbenzoyl) amino]-2,6-dimethylpyridinium hydroxide ;

the internal salt of 1-[(4-chloro-3-sulphamoylbenzoyl) amino]-3-carbamoylpyridinium hydroxide ;

the internal salt of 1-{[4-chloro-2-(2-furylmethylamino)-5-sulphamoylbenzoyl] amino}-2,4,6-trimethylpyridinium hydroxide, and

the internal salt of 1-[(4-chloro-3-sulphamoylbenzoyl) amino]-2,4,6-triphenylpyridinium hydroxyde.

3. A derivative corresponding to general formula II according to Claim 1 selected from :

1-[(4-chloro-3-sulphamoylbenzoyl) amino]-2,4,6-trimethylpyridinium chloride ;

1-{[4-chloro-2-(2-furylmethylamino)-5-sulphamoylbenzoyl] amino}-2,4,6-trimethylpyridinium chloride, and

1-[(4-chloro-3-sulphamoylbenzoyl) amino]-2,4,6-triphenylpyridinium chloride.

4. A process for the preparation of the derivatives corresponding to general formula I according to Claim 1, characterised in that a pyrylium salt corresponding to formula III

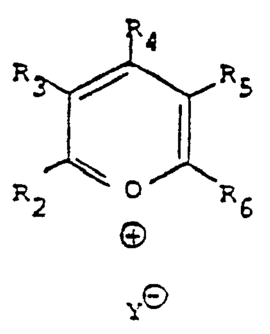

(III)

is reacted with a monosubstituted hydrazine corresponding to formula IV

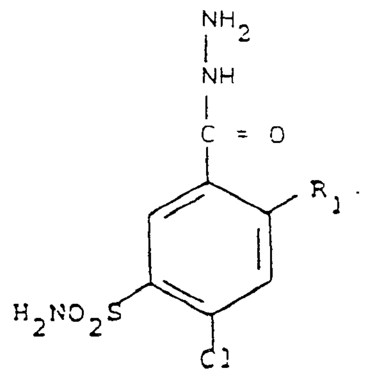

(IV)

in which $R_1$, $R_2$, $R_3$, $R_4$, $R_5$ and $R_6$ have the meanings given in Claim 1, and $Y^{\ominus}$ represents the $BF_4^{\ominus}$, $ClO_4^{\ominus}$ and $CF_3SO_3^{\ominus}$ anions, and in that the corresponding salt obtained is treated with a base so as to form the betaine corresponding to general formula I.

5. A process for preparing the derivatives corresponding to general formula I according to Claim 1, characterised in that a salt of N-aminopyridinium corresponding to general formula V

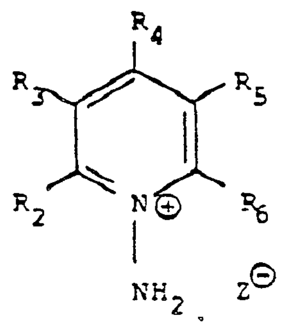

(V)

is reacted with an acid chloride corresponding to general formula VI

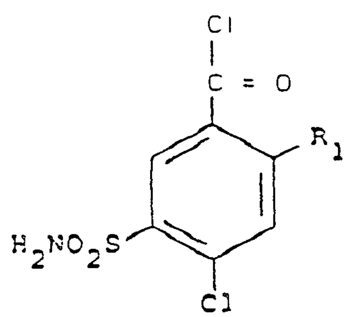

(VI)

In which $R_1$, $R_2$, $R_3$, $R_4$, $R_5$ and $R_6$ have the meanings given in Claim 1 and $Z^{\ominus}$ represents the anions

$Cl^{\ominus}$ , $I^{\ominus}$ and 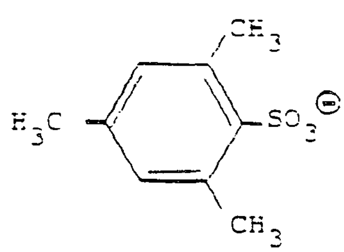

and in that the corresponding betaine of general formula I is obtained in a basic medium.

6. A process for the preparation of the derivatives corresponding to general formula I according to Claim 1, characterised in that N-(2,4-dinitrophenyl)pyridinium chloride corresponding to general formula VII

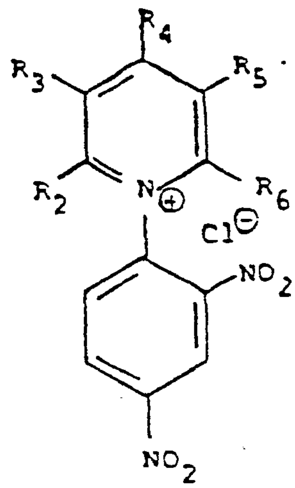

(VII)

is reacted with a monosubstituted hydrazine corresponding to general formula IV

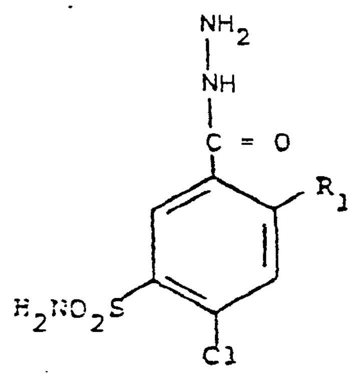

(IV)

in which $R_1$, $R_2$, $R_3$, $R_4$, $R_5$ and $R_6$ have the meanings given in Claim 1, and in that the reaction is carried out in the presence of a tertiary amine used as proton captor for obtaining the corresponding betaines of general formula I.

7. A process for preparing the derivatives corresponding to general formula II according to Claim 1, characterised in that a derivative corresponding to general formula I

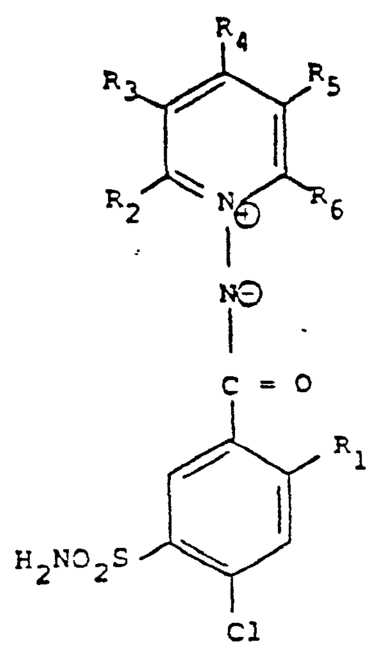

(I)

in which

$R_1$ represents a hydrogen atom, a 2-furyl-methylamino or 2-thenylamino radical and

$R_2$, $R_3$, $R_4$, $R_5$ and $R_6$ are identical or different and represent a hydrogen atom, a $C_1$-$C_4$ linear or branched lower alkyl radical, the phenyl radical, a carbamoyl radical or an amide radical, is reacted with an acid corresponding to formula $H^{\oplus}X^{\ominus}$ within a suitable solvent such as an alcohol or a ketone in order to obtain the corresponding salts of N-aminopyridinium corresponding to general formula II.

8. The derivatives corresponding to general formulae I and II according to one of Claims 1, 2 and 3 as new medicaments.

9. A pharmaceutical composition, characterised in that it contains, in addition to a pharmaceutically acceptable support, at least one derivative corresponding to general formula I or II according to one of Claims 1, 2 and 3.

**Claims** (for the Contracting State AT)

1. A process for preparing derivatives of internal salts of aminopyridinium hydroxide corresponding to general formula I

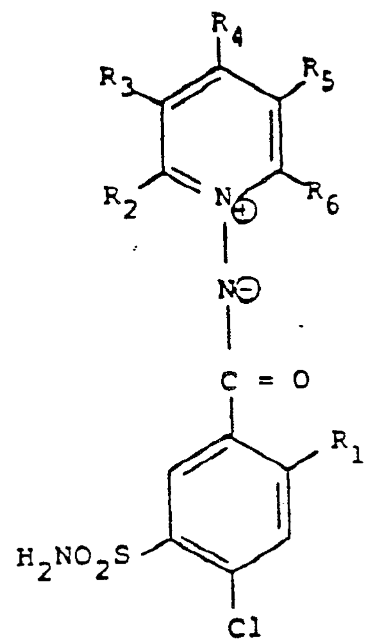

(I)

in which

$R_1$ represents a hydrogen atom, a 2-furyl-methylamino or 2-thenylamino radical and

$R_2$ to $R_6$ are identical or different and represent a hydrogen atom, a $C_1$-$C_4$ linear or branched lower alkyl radical, the phenyl radical, a carbamoyl radical or an amide radical ;

characterised in that a pyrylium salt corresponding to formula III

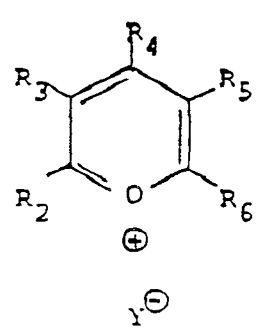

(III)

is reacted with a monosubstituted hydrazine corresponding to formula IV

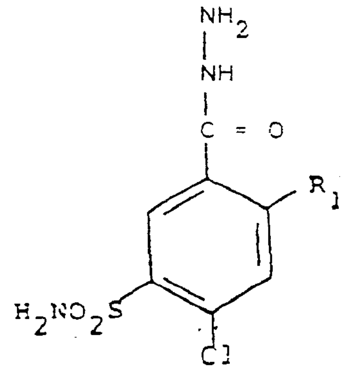

(IV)

in which $R_1$, $R_2$, $R_3$, $R_4$, $R_5$ and $R_6$ have the meanings given above, and $Y^{\ominus}$ represents the $BF_4^{\ominus}$, $ClO_4^{\ominus}$ and $CF_3SO_3^{\ominus}$ anions, and in that the corresponding salt obtained is treated with a base so as to form the betaine corresponding to general formula I.

2. A process for preparing the derivatives corresponding to general formula I according to Claim 1, characterised in that a salt of N-aminopyridinium corresponding to general formula V

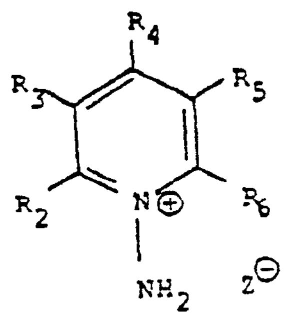

(V)

is reacted with an acid chloride corresponding to general formula VI

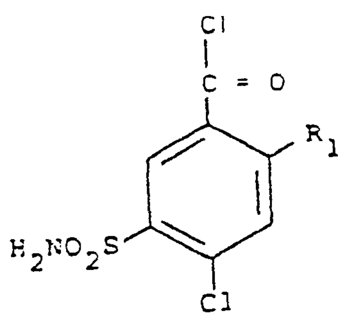

(VI)

in which $R_1$, $R_2$, $R_3$, $R_4$, $R_5$ and $R_6$ have the meanings given in Claim 1 and $Z^{\ominus}$ represents the anions

$Cl^{\ominus}$ , $I^{\ominus}$ and 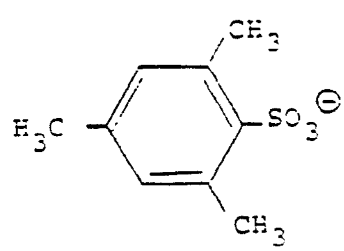

and in that the corresponding betaine of general formula I is obtained in a basic medium.

3. A process for the preparation of the derivatives corresponding to general formula I according to Claim 1, characterised in that N-(2,4-dinitrophenyl)pyridinium chloride corresponding to general formula VII

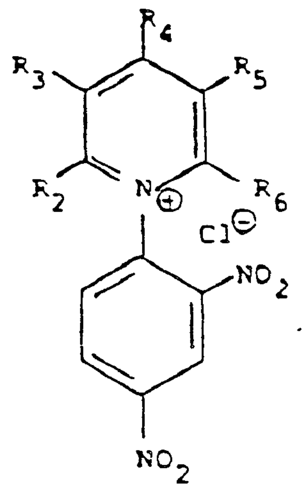

(VII)

is reacted with a monosubstituted hydrazine corresponding to general formula IV

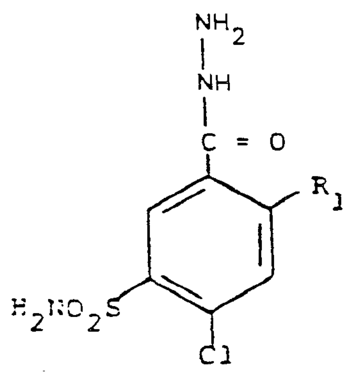

(IV)

in which $R_1$, $R_2$, $R_3$, $R_4$, $R_5$ and $R_6$ have the meanings given in Claim 1, and in that the reaction is carried out in the presence of a tertiary amine used as proton captor for obtaining the corresponding betaines of general formula I.

4. A process for preparing salts corresponding to the derivatives of internal salts of aminopyridinium hydroxide corresponding to general formula II

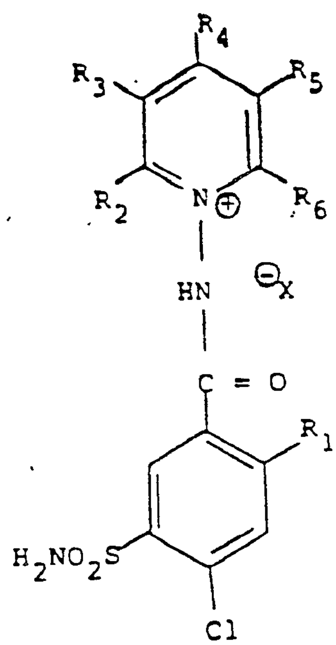

(II)

in which

$R_1$ represents a hydrogen atom, a 2-furyl-methylamino or 2-thenylamino radical and

$R_2$ to $R_6$ are identical or different and represent a hydrogen atom, a $C_1$-$C_4$ linear or branched lower alkyl radical, the phenyl radical, a carbamoyl radical or an amide radical ;

$X^{\ominus}$ represents the anion of a pharmaceutically acceptable acid,

characterised in that the derivative corresponding to general formula I, according to Claim 1, is reacted with an acid corresponding to the formula $H^{\oplus}X^{\ominus}$, within a suitable solvent such as an alcohol or a ketone in order to obtain the corresponding salts of N-aminopyridinium corresponding to general formula II.

5. A process for the preparation of a derivative corresponding to general formula I, according to Claim 1, characterised in that a derivative selected from

the internal salt of 1-[(4-chloro-3-sulphamoylbenzoyl)amino]pyridinium hydroxide ;

the internal salt of 1-[(4-chloro-3-sulphamoylbenzoyl)amino]-2,4,6-trimethylpyridinium hydroxide ;

the internal salt of 1-[(4-chloro-3-sulphamoylbenzoyl)amino]-2,6-dimethylpyridinium hydroxide ;

the internal salt of 1-[(4-chloro-3-sulphamoylbenzoyl)amino]-3-carbamoylpyridinium hydroxide ;

the internal salt of 1-{[4-chloro-2-(2-furylmethylamino)-5-sulphamoylbenzoyl]amino}-2,4,6-trimethylpyridinium hydroxide, and

the internal salt of 1-[(4-chloro-3-sulphamoylbenzoyl)amino]-2,4,6-triphenylpyridinium hydroxide is prepared.

6. A process for preparing the salt of a derivative corresponding to general formula II, according to Claim 4, selected from

1-[(4-chloro-3-sulphamoylbenzoyl)amino]-2,4,6-trimethylpyridinium chloride ;

1-{[4-chloro-2-(2-furylmethylamino)-5-sulphamoylbenzoyl]amino}-2,4,6-trimethylpyridinium chloride, and

1-[(4-chloro-3-sulphamoylbenzoyl)amino]-2,4,6-triphenylpyridinium chloride.

**Patentansprüche** (für die Vertragsstaaten : BE, CH, DE, FR, GB, IT, LI, LU, NL, SE)

1. Derivate innerer Salze von Aminopyridiniumhydroxid sowie deren Salze entsprechend den allgemeinen Formeln :

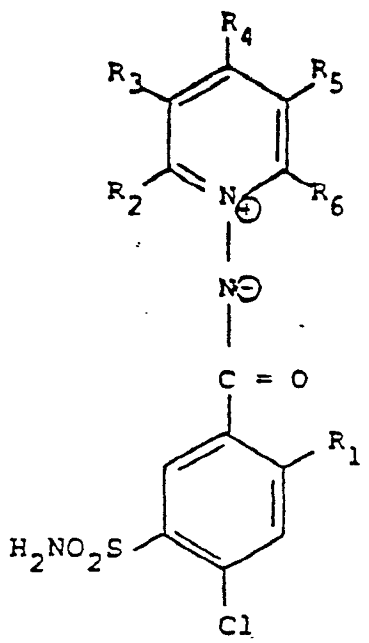

(I)

, und

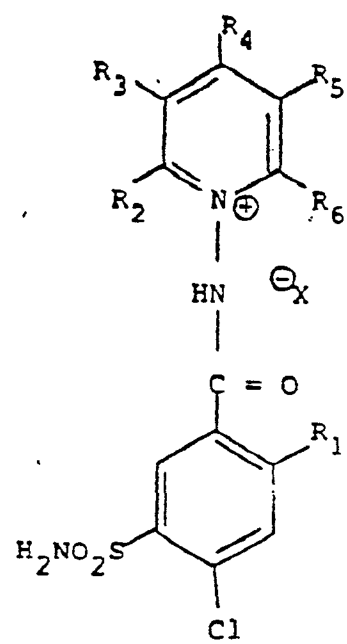

(II)

worin bedeuten :

$R_1$ ein Wasserstoffatom oder eine 2-Furylmethylamino-oder 2-Thenylaminogruppe ;

$R_2$, $R_3$, $R_4$, $R_5$ und $R_6$, die gleich oder verschieden sein können, jeweils ein Wasserstoffatom, eine gerad- oder verzweigtkettige $C_1$- bis $C_4$-Niedrigalkylgruppe, eine Phenylgruppe, eine Carbamoylgruppe oder eine Amidgruppe und

$X^{\ominus}$ das Anion einer pharmazeutisch akzeptablen Säure.

2. Derivat der allgemeinen Formel (I) nach Anspruch 1, dadurch gekennzeichnet, daß es aus dem inneren Salz von

1-[(4-Chlor-3-sulfamoylbenzoyl)-amino]-pyridiniumhydroxid,

1-[(4-Chlor-3-sulfamoylbenzoyl)-amino]-2,4,6-trimethylpyridiniumhydroxid,

1-[(4-Chlor-3-sulfamoylbenzoyl)-amino]-2,6-dimethylpyridiniumhydroxid,

1-[(4-Chlor-3-sulfamoylbenzoyl)-amino]-3-carbamoylpyridiniumhydroxid,

1-{[4-Chlor-2-(2-furylmethylamino)-5-sulfamoylbenzoyl]-amino}-2,4,6-trimethylpyridiniumhydroxid oder

1-[(4-Chlor-3-sulfamoylbenzoyl)-amino]-2,4,6-triphenylpyridiniumhydroxid besteht.

3. Derivat der allgemeinen Formel (II) nach Anspruch 1, dadurch gekennzeichnet, daß es aus

1-[(4-Chlor-3-sulfamoylbenzoyl)-amino]-2,4,6-trimethylpyridiniumchlorid,

1-{[4-Chlor-2-(2-furylmethylamino)-5-sulfamoylbenzoyl]-amino}-2,4,6-trimethylpyridiniumchlorid oder

1-[(4-Chlor-3-sulfamoylbenzoyl)-amino]-2,4,6-triphenylpyridiniumchlorid besteht.

4. Verfahren zur Herstellung der Derivate der allgemeinen Formel (I) nach Anspruch 1, dadurch gekennzeichnet, daß man ein Pyriliumsalz der Formel :

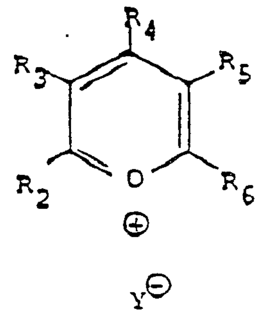

(III)

worin $R_2$, $R_3$, $R_4$, $R_5$ und $R_6$ die in Anspruch 1 angebene Bedeutung besitzen und $Y^\ominus$ für die Anionen $BF_4^\ominus$, $ClO_4^\ominus$ oder $CF_3SO_3^\ominus$ steht, mit einem einfachsubstituierten Hydrazin der Formel :

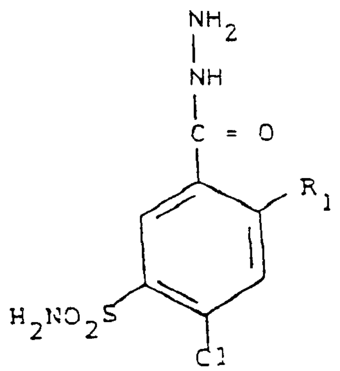

(IV)

worin $R_1$ die in Anspruch 1 angegebene Bedeutung besitzt, reagieren läßt und das erhaltene entsprechende Salz zur Bildung des Betains der allgemeinen Formel (I) mit einer Base behandelt.

5. Verfahren zur Herstellung der Derivate der allgemeinen Formel (I) nach Anspruch 1, dadurch gekennzeichnet, daß man ein N-Aminopyridiniumsalz der allgemeinen Formel :

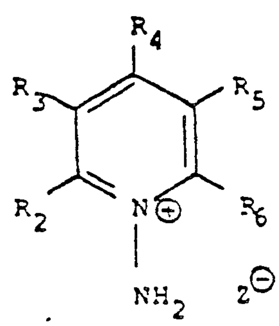

(V)

worin $R_1$, $R_2$, $R_3$, $R_4$, $R_5$ und $R_6$ die in Anspruch 1 angegebene Bedeutung besitzen und $Z^\ominus$ für die Anionen $Cl^\ominus$, $J^\ominus$ oder

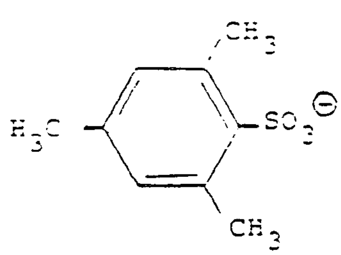

steht, mit einem Säurechlorid der allgemeinen Formel :

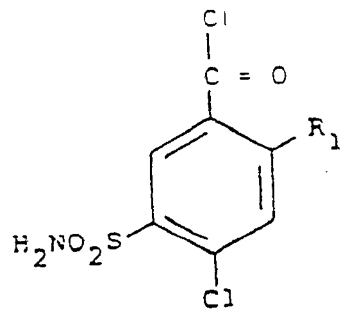

(VI)

worin $R_1$ die in Anspruch 1 angegebene Bedeutung besitzt, reagieren läßt und in alkalischem Milieu das der allgemeinen Formel (I) entsprechende Betain gewinnt.

6. Verfahren zur Herstellung der Derivate der allgemeinen Formel (I) nach Anspruch 1, dadurch gekennzeichnet, daß man das N-(2,4-Dinitrophenyl)-pyridiniumchlorid der allgemeinen Formel :

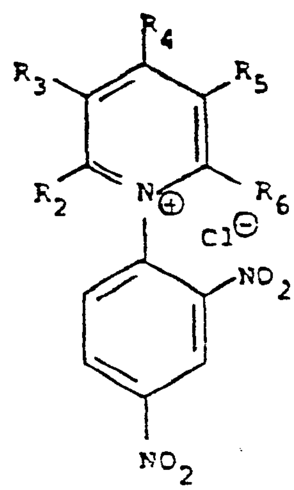

(VII)

worin $R_2$, $R_3$, $R_4$, $R_5$ und $R_6$ die in Anspruch 1 angegebene Bedeutung besitzen, mit einem einfachsubstituierten Hydrazin der allgemeinen Formel :

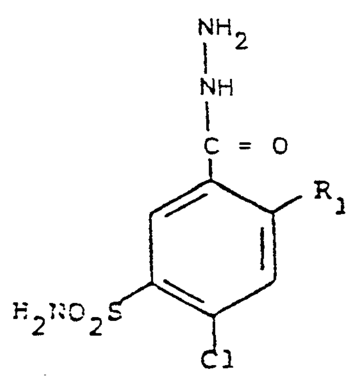

(IV)

worin $R_1$ die in Anspruch 1 angegebene Bedeutung besitzt, reagieren läßt und die Umsetzung in Gegenwart eines als Protonenfänger dienenden tertiären Amins ablaufen läßt, um die der allgemeinen Formel (I) entsprechenden Betaine herzustellen.

7. Verfahren zur Herstellung der Derivate der allgemeinen Formel (II) nach Anspruch 1, dadurch gekennzeichnet, daß man ein Derivat der allgemeinen Formel :

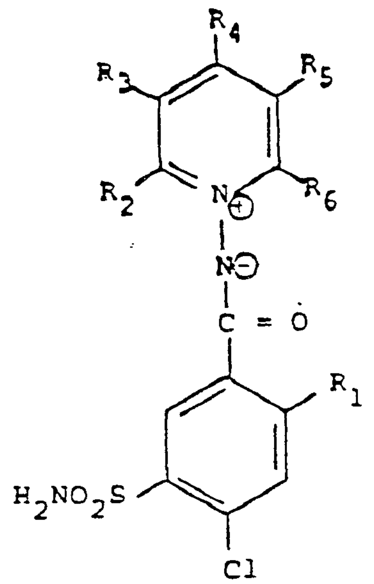

(I)

worin bedeuten :

$R_1$ ein Wasserstoffatom oder eine 2-Furylmethylamino- oder 2-Thenylaminogruppe und

$R_2$, $R_3$, $R_4$, $R_5$ und $R_6$, die gleich oder verschieden sein können, jeweils ein Wasserstoffatom, eine gerad-oder verzweigtkettige $C_1$- bis $C_4$-Niedrigalkylgruppe, eine Phenylgruppe, eine Carbamoylgruppe oder eine Amidgruppe,

in einem geeigneten Lösungsmittel, wie einem Alkohol oder Keton mit einer Säure der Formel $H^{\oplus}X^{\ominus}$ reagieren läßt, um die entsprechenden N-Aminopyridiniumsalze der allgemeinen Formel (II) herzustellen.

8. Als neue Medikamente die Derivate der allgemeinen Formeln (I) und (II) nach einem der Ansprüche 1, 2 und 3.

9. Arzneimittel, dadurch gekennzeichnet, daß es außer einem pharmazeutisch akzeptablen Träger mindestens ein Derivat der allgemeinen Formeln (I) oder (II) nach einem der Ansprüche 1, 2 oder 3 enthält.

**Patentansprüche** (für den Vertragsstaat AT)

1. Verfahren zur Herstellung von Derivaten innerer Salze von Aminopyridiniumhydroxid der allgemeinen Formel :

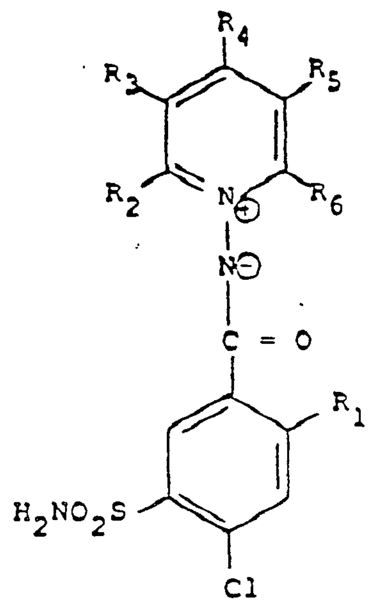

(I)

worin bedeuten :

$R_1$ ein Wasserstoffatom oder eine 2-Furylmethylamino-oder 2-Thenylaminogruppe und

$R_2$ bis $R_6$, die gleich oder verschieden sein können, jeweils ein Wasserstoffatom, eine gerad- oder verzweigtkettige $C_{1-4}$-Niedrigalkylgruppe, eine Phenylgruppe, eine Carbamoylgruppe oder eine Amidgruppe,

dadurch gekennzeichnet, daß man ein Pyriliumsalz der Formel :

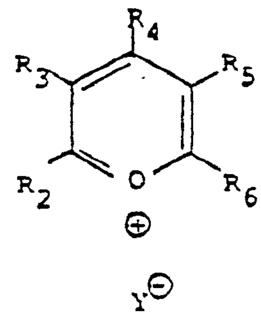

(III)

worin $R_2$, $R_3$, $R_4$, $R_5$ und $R_6$ die oben angegebene Bedeutung besitzen, und $Y^{\ominus}$ für die Anionen $BF_4^{\ominus}$, $ClO_4^{\ominus}$ oder $CF_3SO_3^{\ominus}$ steht, mit einem einfachsubstituierten Hydrazin der Formel :

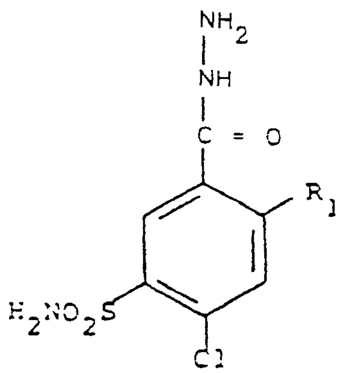

(IV)

worin $R_1$ die oben angegebene Bedeutung besitzt, reagieren läßt und das erhaltene entsprechende Salz zur Bildung des Betains der allgemeinen Formel (I) mit einer Base behandelt.

2. Verfahren zur Herstellung von Derivaten der allgemeinen Formel (I) nach Anspruch 1, dadurch gekennzeichnet, daß man ein N-Aminopyridiniumsalz der allgemeinen Formel :

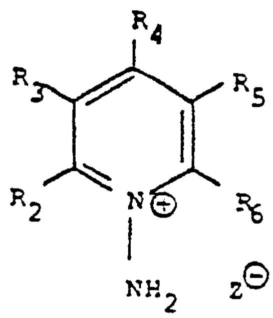

(V)

worin $R_2$, $R_3$, $R_4$, $R_5$ und $R_6$ die in Anspruch 1 angegebene Bedeutung besitzen und $Z^{\ominus}$ für die Anionen $Cl^{\ominus}$, $J^{\ominus}$ oder

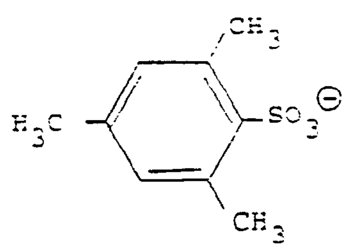

steht, mit einem Säurechlorid der allgemeinen Formel :

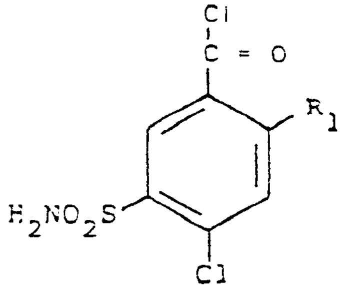

(VI)

worin $R_1$ die in Anspruch 1 angegebene Bedeutung besitzt, reagieren läßt und in alkalischem Milieu das der allgemeinen Formel (I) entsprechende Betain gewinnt.

3. Verfahren zur Herstellung von Derivaten der allgemeinen Formel (I) nach Anspruch 1, dadurch gekennzeichnet, daß man das N-(2,4-Dinitrophenyl)-pyridiniumchlorid der allgemeinen Formel :

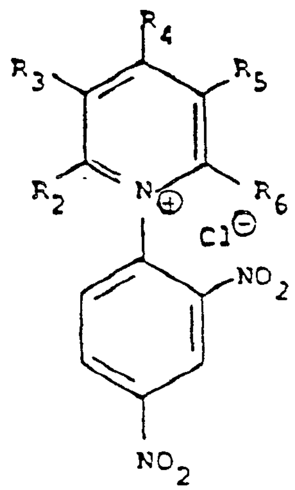

(VII)

worin $R_2$, $R_3$, $R_4$, $R_5$ und $R_6$ die in Anspruch 1 angegebene Bedeutung besitzen, mit einem einfachsubstituierten Hydrazin der allgemeinen Formel :

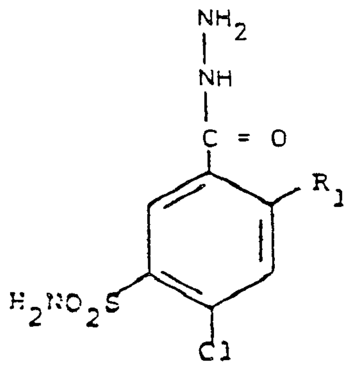

(IV)

worin $R_1$ die in Anspruch 1 angegebene Bedeutung besitzt, reagieren läßt und die Umsetzung in Gegenwart eines als Protonenfänger dienenden tertiären Amins ablaufen läßt, um die der allgemeinen Formel (I) entsprechenden Betaine herzustellen.

4. Verfahren zur Herstellung von Derivaten innerer Salze von Aminopyridiniumhydroxid entsprechenden Salzen der allgemeinen Formel :

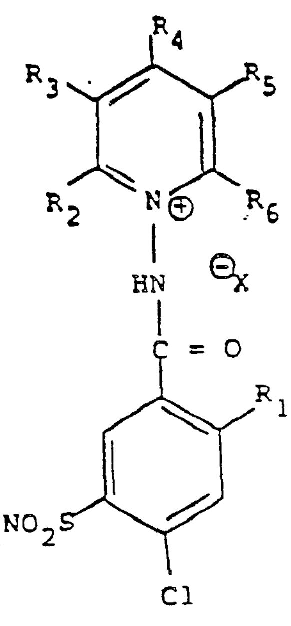

(II)

worin bedeuten :

$R_1$ ein Wasserstoffatom oder eine 2-Furylmethylamino-oder 2-Thenylaminogruppe,

$R_2$, $R_3$, $R_4$, $R_5$ und $R_6$, die gleich oder verschieden sein können, jeweils ein Wasserstoffatom, eine gerad- oder verzweigtkettige $C_{1-4}$-Niedrigalkylgruppe, eine Phenylgruppe, eine Carbamoylgruppe oder eine Amidgruppe und

$X^{\ominus}$ das Anion einer pharmazeutisch akzeptablen Säure, dadurch gekennzeichnet, daß man ein Derivat der allgemeinen Formel (I) nach Anspruch 1 in einem geeigneten Lösungsmittel, wie einem Alkohol oder Keton, mit einer Säure der Formel $H^{\oplus}$ $X^{\ominus}$ reagieren läßt, um die entsprechenden N-Aminopyridiniumsalze der allgemeinen Formel (II) herzustellen.

5. Verfahren zur Herstellung eines Derivats der allgemeinen Formel (I) nach Anspruch 1, dadurch gekennzeichnet, daß man das innere Salz von

1-[(4-Chlor-3-sulfamoylbenzoyl)-amino]-pyridiniumhydroxid,

1-[(4-Chlor-3-sulfamoylbenzoyl)-amino]-2,4,6-trimethylpyridiniumhydroxid,

1-[(4-Chlor-3-sulfamoylbenzoyl)-amino]-2,6-dimethylpyridiniumhydroxid,

1-[(4-Chlor-3-sulfamoylbenzoyl)-amino]-3-carbamoylpyridiniumhydroxid,

1-{[4-Chlor-2-(2-furylmethylamino)-5-sulfamoylbenzoyl]-amino}-2,4,6-trimethylpyridiniumhydroxid oder

1-[(4-Chlor-3-sulfamoylbenzoyl)-amino]-2,4,6-triphenylpyridiniumhydroxid herstellt.

6. Verfahren zur Herstellung eines Salzes eines Derivats der allgemeinen Formel (II) nach Anspruch 4, dadurch gekennzeichnet, daß man

1-[(4-Chlor-3-sulfamoylbenzoyl)-amino]-2,4,6-trimethylpyridiniumchlorid,

1-{[4-Chlor-2-(2-furylmethylamino)-5-sulfamoylbenzoyl]-amino}-2,4,6-trimethylpyridiniumchlorid oder

1-[(4-Chlor-3-sulfamoylbenzoyl)-amino]-2,4,6-triphenylpyridiniumchlorid herstellt.